# EUROPEAN PATENT APPLICATION

(11) **EP 1 886 627 A1**
(43) Date of publication of application: **13.02.2008**
(21) Application number: 07015623.7
(22) Date of filing: 08.08.2007
(51) Int. Cl.: A61B 5/055

(54) **Magnetic resonance imaging system, a gradient coil, and a method of using the system**

(30) Priority: 08.08.2006 US 500703
(71) Applicant: Philips Medical Systems MR, Inc., Latham NY 12110 (US)
(72) Inventor: Jonas, Philip A., Delmar NY 12054 (US); Molyneaux, David A., Micanopy FL 32667 (US); Reis, Chandra T., Altamont NY 12009 (US)
(74) Representative: Zimmermann, Gerd Heinrich

(57) **Abstract**

A cylindrical MRI system can be configured such that a patient does not have to be in a lying position during analysis. In a particular embodiment, the patient can sit during analysis. The cylindrical MRI system (100) can be oriented such that a central axis is not parallel to the floor, and in one embodiment, is substantially perpendicular to the floor. In one embodiment, the cylindrical MRI system can be configured to allow an object to contact the patient when the patient is within the analyzing region, even when the primary magnet (102; 202) is at field. In another aspect, an MRI system can include a primary magnet and a gradient coil (104; 304; 404) with different types of shapes. In still another aspect, an MRI system can include a gradient coil that includes a combination of portions having different shapes.

## Description

The present disclosure is related to United States Patent Application Number 11/500,703 entitled "Magnetic resonance imaging system, a gradient coil, and a method of using the system" by Jonas et al. filed on August 8, 2006, which is assigned to the current assignee hereof and incorporated herein by reference in its entirety.

The disclosure relates to systems, magnets, and methods, and more particularly to magnetic resonance imaging systems, gradient coils, and methods of using the systems.

Magnetic resonance imaging ("MRI") systems are widely used for analyzing patients. MRI systems typically have one of two types of designs. A cylindrical MRI system includes an analyzing region that lies within an annulus of a primary magnet. Conventionally, the central axis of the annulus lies along a line that is parallel to the floor. For a cylindrical MRI system, the patient lies on a supporting member as the patent is inserted, analyzed, and removed from the analyzing region. An open MRI system includes a pair of spaced-apart, primary magnets, wherein the analyzing region lies between the primary magnets. The open MRI system may be oriented such that one primary magnet overlies the other primary magnet. Alternatively, the open MRI system may be oriented with the primary magnets side-by-side.

Substantially any region of a patient can be analyzed, including the pelvic region. When analyzing the pelvic region of a patient using a conventional MRI system, more than half, and sometimes nearly all, of the patient is placed within the annulus of the primary magnet (cylindrical MRI system) or between the primary magnets (open MRI system). Many patients can become claustrophobic when being analyzed, particularly when analyzing the pelvic region. Such patients may have anxiety attacks and require sedation. The procedure is unpleasant for the patients.

The conventional MRI systems are large. Each of the primary magnets used in MRI systems can have a diameter that is at least approximately 1.2 meters (approximately 4.0 feet). In addition, the conventional MRI systems occupy a substantial amount of floor space within a hospital or other medical facility. The floor space is expensive, and the MRI system by itself can occupy over approximately 2.3 m² (over approximately 25 ft²).
In light of the above, a magnetic resonance system according to independent claim 1, and a method of using a magnetic resonance system according to independent claim 7 are provided.
Further advantages, features, aspects and details of the invention are evident from the dependent claims, the description and the drawings.

The present disclosure may be better understood, and its numerous features and advantages made apparent to those skilled in the art, by referencing the accompanying drawings.

FIG. 1 includes an illustration of an MRI system, a chair including a seat, and a patient in the chair.

FIG. 2 includes an illustration of a perspective view of a primary magnet.

FIG. 3 includes an illustration of a perspective view of a gradient coil.

FIG. 4 includes an illustration of a cross-sectional view of another gradient coil.

FIG. 5 includes a flow diagram of a method of using an MRI system.

The use of the same reference symbols in different drawings indicates similar or identical items. Skilled artisans will appreciate that elements in the figures are illustrated for simplicity and clarity and have not necessarily been drawn to scale.

### DETAILED DESCRIPTION

A cylindrical MRI system can be configured such that a patient does not have to be in a lying position during analysis. In a particular embodiment, the patient can sit during analysis. The cylindrical MRI system can be oriented such that a central axis is not parallel to the floor, and in one embodiment, is substantially perpendicular to the floor. In one embodiment, the cylindrical MRI system can be configured to allow an object to contact the patient when the patient is within the analyzing region, even when the primary magnet is at field. In another aspect, an MRI system can include a primary magnet and a gradient coil with different types of shapes. In still another aspect, an MRI system can include a gradient coil that includes a combination of portions having different shapes. The MRI systems described herein can reduce the amount of required floor space occupied by the MRI system and improve the patient experience.

In a first aspect, a magnetic resonance imaging system can include a primary magnet, wherein an opening extends through a center of the primary magnet. The magnetic resonance imaging system can also include an analyzing region disposed within the opening, wherein the analyzing region is configured to allow a patient to sit within the magnetic resonance imaging system when the patient is being analyzed.

In a second aspect, a method of using a magnetic resonance imaging system can include disposing a patient within the magnetic resonance imaging system, wherein the magnetic resonance imaging system includes a primary magnet having an annulus, and an opening disposed within the annulus. The method can also include analyzing the patient using the magnetic resonance imaging system, wherein analyzing is performed when the patient is disposed within the opening and not in a lying position.

In a third aspect, a magnetic resonance imaging system can include a primary magnet having a first type of shape and a gradient coil having a second type of shape different from the first type of shape.

In a fourth aspect, a gradient coil for a magnetic resonance imaging system can include a substantially cylindrical portion including an annulus and a first opening within the annulus, and a substantially flat portion adjacent to the substantially cylindrical portion, wherein the substantially flat portion is disposed along a bottom of the first opening.

A few terms are defined or clarified to aid in understanding of the terms as used throughout this specification.

The term "analyzing region," with respect to an MRI system, is intended to mean a region within the MRI system where a patient or other object can be properly analyzed when using the MRI system.

The term "chair" is intended to mean a structure or other object used to support the back and legs of patient when the patient would be in a sitting position.

The term "cylindrical MRI system" is intended to mean an MRI system that has a primary magnet that surrounds a patient or other object when analyzed using the MRI system.

The terms "gradient coil" is intended to mean a coil or a set of coils that can be used during analysis of a patient to affect a magnetic field generated by a primary magnet. Typically, as is common in the art, "gradient coil" refers to any system that creates a time-varying modulation of the primary magnetic field for a purpose of focusing data collection on a particular location.

As used in this specification, an intersection of a line and a plane is expressed in terms of acute angles only. Thus, the maximum value of an angle at the intersection of a line and plane is 90°. An intersection of two lines can acute or obtuse. Thus, the maximum value of an angle at the intersection of two lines is 180°.

The term "open MRI system" is intended to mean an MRI system that has a pair of primary magnets that lie on opposite sides of a patient or other object when analyzed using the MRI system.

The term "pass-through object" is intended to mean tubing, a sensor, a probe, a scalpel, a syringe, a biopsy needle, or other instrument used in analyzing, diagnosing, treating, or performing a medical procedure on a patient, wherein at least a portion of such tubing, sensor, probe, scalpel, syringe, biopsy needle, or other instrument is capable of being passed or extending through an opening in a magnetic resonance imaging system while a primary magnet of the system is at a magnetic field substantially greater than 0.0 T.

The term "pelvic region" is intended to mean a region of a patient that extends from a plane substantially perpendicular to length of the patient and including the top of a hip bone (iliac crest of the ilium) to another plane substantially perpendicular to length of the patient and including the bottom of a bone adjacent to the public arch (ischium).

The term "primary magnet" is intended to mean a magnet capable of generating a substantially static magnetic field that is significantly stronger than a gradient coil.

The term "seat" is intended to mean a structure or other object used to support the buttocks of a patient, the pelvic region of a patient, or a combination thereof when the patient would be in a sitting position.

The term "typical operating state" is intended to mean a state in which all superconducting elements along a superconducting current path are in their superconducting states.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Additionally, for clarity purposes and to give a general sense of the scope of the embodiments described herein, the use of the "a" or "an" is employed to describe one or more articles to which "a" or "an" refers. Therefore, the description should be read to include one or at least one whenever "a" or "an" is used, and the singular also includes the plural unless it is clear that the contrary is meant otherwise.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

To the extent not described herein, many details regarding specific materials, processing acts, and components, assemblies, and systems are conventional and may be found in textbooks and other sources within the superconducting, cryogenic, and medical device arts.

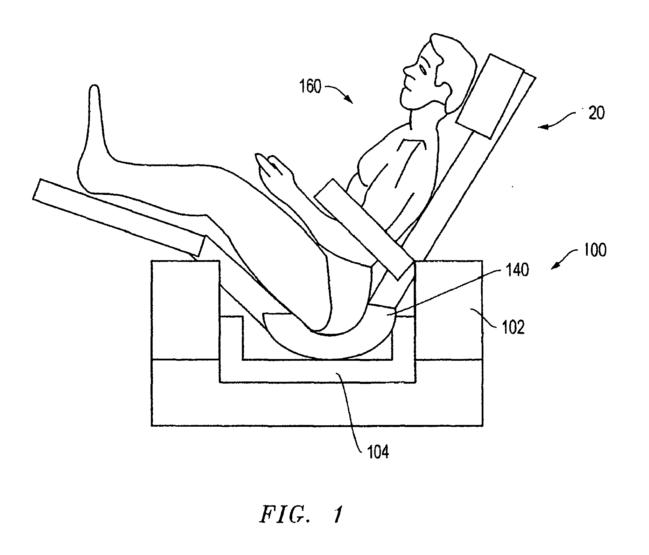

FIG. 1 includes an illustration of an MRI system 100 when analyzing a patient 160. In embodiment as illustrated in FIG. 1, the MRI system 100 includes a cylindrical MRI system. The MRI system 100 includes a primary magnet section 102 and a gradient coil section 104, both of which are described in more detail below. FIG. 1 also includes a chair 120, a seat 140, and the patient 160 disposed within the MRI system 100. The chair 120 and seat 140 may or may not be part of the MRI system 100. Details, design and configuration options of chairs and seats are described in more detail in United States Patent Application Number 11/500,724 entitled "Seat, a Chair Including a Seat, And a Method of Using a Magnetic Resonance Imaging System Including a Seat" by Jonas et al. filed on August 8, 2006. After reading this specification, skilled artisans will appreciate that another seat or chair may be used or potentially no seat or chair may be used.

The primary magnet section 102 can generate a relatively large, substantially static magnetic field when the MRI system 100 is in its typical operating state. In one embodiment when the MRI system 100 is in its typical operating state, the magnetic field generated by a primary magnet is significantly greater than 0.0 T and can be at least 0.1, 0.5, 0.9, or 1.5 T, and in another embodiment, the magnetic field may not exceed 10, 5, or 3 T. In still other embodiments, a stronger or weaker magnetic field may be used with the primary magnet section 102.

The primary magnet section 102 can include a permanent magnet or an electromagnetic coil. The primary magnet can be a solenoid that includes a superconducting or other conductive wire. In one embodiment, the primary magnet section 102 can include one primary magnet, and in another embodiment can include more than one primary magnet. When the primary magnet section 102 includes a plurality of primary magnets, the primary magnets can be oriented such that primary magnets lie along a central axis. For simplicity, the remainder of the description of the primary magnet section 102 will refer to a primary magnet, even though one or more than one primary magnet may be present.

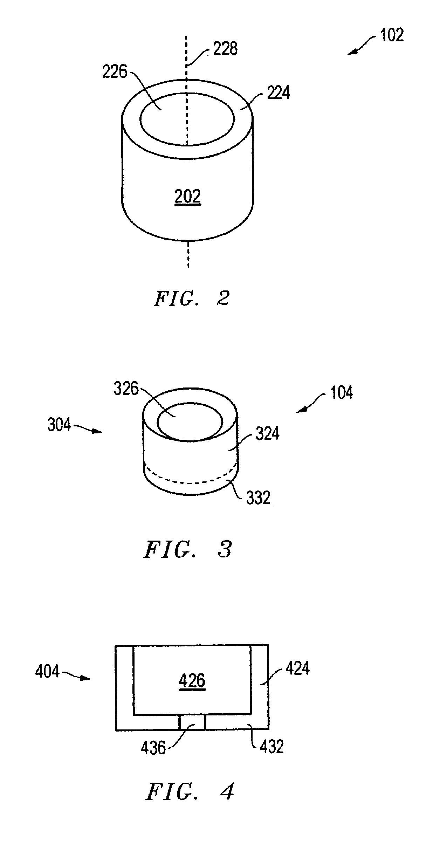

FIG. 2 includes a perspective view of the primary magnet 202 from the primary magnet section 102. The primary magnet 202 can be in the shape of an annulus 224 that defines an opening 226 that extends along the center of the primary magnet 202 and the inside of the annulus 224. An analyzing region for the MRI system 100 is disposed within the opening 226. The analyzing region can have a shape similar to the opening 226. In a particular embodiment, the analyzing region is shorter than the opening 226.

A central axis 228 extends through the opening 226 and analyzing region. Unlike a conventional cylindrical MRI system that has a central axis that lies along a line parallel to a plane that corresponds to the floor over which the MRI system 100 lies, the central axis 228 of the MRI system 100 lies along a line that is not substantially perpendicular to the plane. In one embodiment, the line and the plane intersect at an angle of at least 30°, 45°, or 60°. In a particular embodiment, the line is substantially perpendicular to the plane. In still another embodiment, the angle can be less than 90°, 80°, or 70°. In further embodiments, intermediate values for the angles (e.g., between 80° and 90°) can be used. After reading this specification, skilled artisans will appreciate that angles less than 30° can be used. The orientation of the primary magnet 202 and analyzing region of the MRI system 100 can allow a patient to sit within the analyzing region when being analyzed. Methods of using the MRI system 100 are described later in this specification.

The gradient coil section 104 can include a meander path or an electromagnetic coil that may include a superconducting or other conductive wire, or any other construction used in the art to create a time-varying modulation of the primary magnetic field for the purposes of focusing the data collection on a particular location. In one embodiment, the gradient coil section 104 can include one gradient coil, and in another embodiment can include more than one gradient coil. For simplicity, the remainder of the description of the gradient coil section 104 will refer to a gradient coil, even though one coil or more than one coil (e.g., a set of coils) may be present.

The gradient coil can be a cylindrical style, pancake style, or a combination that includes a cylindrical-shaped portion and a substantially flat portion. FIG. 3 includes a perspective view of the gradient coil 304 from the gradient coil section 104. In the embodiment as illustrated in FIG. 3, the gradient coil 304 includes a cylindrical-shaped portion 324, having an opening 326, and a pancake style portion 332, which is a substantially flat portion. The intersection of the two portions is illustrated by the dashed line in FIG. 3. The two portions may be formed from a single piece of material or may include different pieces adjacent to each other. In a particular embodiment, the cylindrical-shaped portion 324 and the pancake style portion 332 abut each other, and in another embodiment (not illustrated) may be spaced apart by a relatively small distance. In one embodiment, the pancake style portion 332 is substantially solid and does not include an opening extending therethrough.

FIG. 4 includes an illustration of a cross-sectional view of another embodiment. The gradient coil 404 includes a cylindrical-shaped portion 424 and a pancake style portion 432, which is a substantially flat portion. The cylindrical-shaped portion 424 includes an opening 426, and the pancake style portion 432 is not substantially solid and includes an opening 436. In one embodiment, the cylindrical-shaped portion 424 is in the form of an annulus having an outer radius of the annulus and an inner radius, which defines the opening 436. The width of the opening 426 is larger than the width of the opening 436. In another embodiment, the outer radius of the annulus of cylindrical-shaped portion 424 may be no greater than 3 times the inner radius of the annulus, no greater than inner radius, no greater than 0.5 times the inner radius, or smaller. In another embodiment, a width of the pancake style portion 432 at an outer perimeter is more than 1.5 times a width of the opening 436, more than 3 times the width of the opening 436, more than 5 times the width of the opening 436, or larger. After reading this specification, skilled artisans will appreciate that different values can be used. An object (e.g., a probe, a tube, a scalpel, an implant, etc.) can be inserted into the opening 426, opening 436, or both.

In one embodiment, the MRI system 100 includes a primary magnet having a first type of shape, and a gradient coil having a second type of shape different from the first type of shape. Conventionally, a cylindrical-shaped gradient coil is used with a cylindrical-shaped primary magnet, and pancake-shaped gradient coils are used in open MRI systems. Unlike a conventional MRI system, the MRI system 100 can use a pancake-shaped gradient coil with the primary magnet 202. In another embodiment (not illustrated), an open MRI system can use a cylindrical-shaped gradient coil. In still another embodiment, a polygon-shaped gradient coil may be used with a cylindrical MRI system or an open MRI system.

The MRI system 100 can include another section not illustrated. For example, the MRI system 100 can include a vessel that contains a cryogenic liquid, electrical and electronic sub-systems for operating the MRI system 100, magnetic field shielding (active, passive, or a combination thereof), another suitable sub-system, or any combination thereof.

The MRI systems described herein can be significantly smaller than commercial NIRI systems currently in use. The MRI system 100 may occupy less than approximately 2.2 m² (less than approximately 24 ft²) in one embodiment, less than approximately 2.0 m² (less than approximately 20 ft²) in another embodiment, less than approximately 1.5 m² (less than approximately 17 ft²) in still another embodiment, or even less floor space. In one embodiment, the MRI system is configured such that it has a single opening for the ingress and egress of the patient.

Due in part to their smaller size, the MRI systems described herein can have a substantially equal or significantly higher primary magnetic field in the region to be imaged, as compared to a conventional MRI system. Higher magnetic fields give images with higher resolution. For example, the MRI system 100 may have a primary magnetic field of 1.5 Tesla in one embodiment, greater than approximately 3T in another embodiment, greater than approximately 5T in still another embodiment, or even higher magnetic field.

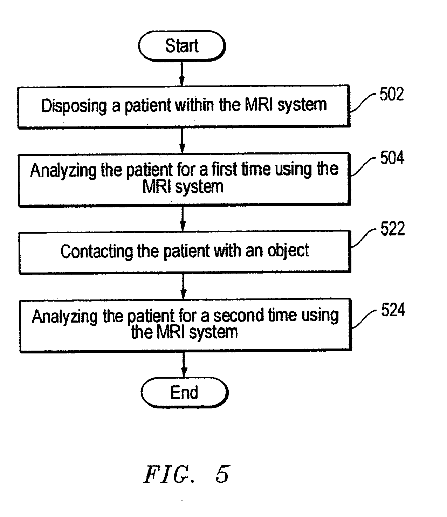

Attention is now directed to methods of using the MRI system 100. Any of the previously described embodiments may be used. The method can include disposing a patient within the MRI system, at block 502 in FIG. 5. In one embodiment, the chair and seat may already be positioned within the MRI system. In a particular embodiment, the patient may sit in the chair without any assistance, and in another embodiment, the patient may be assisted. Such assistance may be provided mechanically. In another embodiment, a human may assist the patient into the chair. In still another embodiment, the patient may be on the seat and the combination of the patient and the seat can be lowered into the opening of the MRI system 100. In a further embodiment, a chair, a seat, or both may not be used. The primary magnet section 102 may include padding or have padding attached to it. In a particular embodiment, the pelvic region of the patient may be unsupported. Thus, disposing should be construed broadly and may be performed by the patient without any assistance or may include assistance and may or may not include a chair, a seat, or both.

In any one or more of the embodiments where a seat is used, the method can optionally include positioning the patient onto the seat. The positioning may be performed before disposing a patient within the MRI system, during disposing a patient within the MRI system, after disposing a patient within the MRI system, or any combination thereof. The position may be performed by the patient with or without assistance. Positioning may be performed to place the patient in proper alignment with sensors in the chair, seat, or both, to improve comfort of the patient, or another reason.

The method can also include analyzing the patient for a first time using the MRI system, at block 504. Analyzing can be performed when the patient is in a sitting position. In a particular embodiment, a head and a leg of the patient extend outside of an analyzing region of the MRI system. A first line can be defined to include a first point corresponding to a center of the pelvic region of the patient and a second point corresponding to a center of the torso at the base of the neck of the patient. A second line can be defined to include the first point and a third point corresponding to a center of a knee of the patient. An angle is formed at an intersection of the first line and the second line may be no greater than 120° in one embodiment, no greater than 110° in another embodiment, and no greater than 100° in still another embodiment. The angle may be at least 60° in one embodiment and at least 80° in another embodiment. In another embodiment, a different angle may be used that is less than 60°, greater than 120°, or has another value between 60° and 120° (e.g., 90°). In yet another embodiment, the angle can be changed, such that during a first portion of the analysis, one angle is used, and during a second portion of analysis, a different angle is used. After reading this specification, skilled artisans will be able to select an angle that meets their needs or desires.

The method can optionally include contacting the patient with an object, at block 522. The object can be a pass-through object. In one embodiment, the contacting can be performed before, during, or after images are taken using the MRI system. In a particular embodiment, the contacting can be performed when the patient is within the analyzing region of the MRI system and when the MRI system is in its typical operating state. The primary magnet section is at a magnetic field significantly greater than 0.0 T, and typically is within a specified operating range. In one embodiment, the contacting can include inserting a probe into a cavity of the patient. In another embodiment, a scalpel, laser, or other tool can be used to remove or oblate a cancerous growth or other matter from the patient. In still another embodiment, the object can be an implant or other article to be inserted into the patient. In still another embodiment, the object can be used to perform a desired surgery. An opening within the gradient coil can allow such contact. For example, the gradient coil may be cylindrical or otherwise include an opening, such as the gradient coil 404 in FIG. 4, which includes opening 436. If the patient is on a seat or chair, the seat or chair may likewise include an opening to allow the object to contact the patient. When the MRI system is configured to allow the optional contact, the patient can be analyzed, diagnosed, treated, or any combination thereof without the patient having to be moved out of and back into the analyzing region of the MRI system. Although not required, a patient may be removed from the analyzing region of the MRI system when contacting the patient with the object or during part of the contacting period (e.g., the patient is re-disposed within the analyzing region after contacting starts but before it ends).

The method can still further include analyzing the patient for a second time using the MRI system, at block 524 in FIG. 5. The analysis for the second time is optional. In one embodiment, the analysis for the second time may be performed after repositioning the patient within the analyzing region. In another embodiment, the analysis for the second time may be performed after contacting the patient with the object. If the object includes a sensor, the analysis for the second time may be performed to obtain different data. If the object includes a scalpel, laser, or other tool, the analysis for the second time may be performed to confirm that the treatment or other medical procedure was successful, properly completed, or the like.

Analyzing the patient for the second time may be performed using any one or more of the embodiments previously described with respect to analyzing the patient for the first time. The actual embodiments used in analysis of the patient for the first and second times may be the same or different.

The MRI systems and methods of using them as described herein can allow for a patient to be in a sitting position when being analyzed, diagnosed, treated, having another medical procedure performed, or any combination thereof. During analyzing the patient, the head and a leg of the patient extend outside of a same side of the opening 226 within the annulus 224 of the primary magnet. The ability to sit can allow the patient to be more comfortable. Additionally, the patient may have a better MRI experience. The head of the patient does not need to enter or pass through the opening in the primary magnet section. Thus, the patient is less likely to feel claustrophobic when the pelvic region of the patient is disposed within the analyzing region of the MRI system. Thus, the patient may be less anxious and less likely to require sedation.

Many different aspects and embodiments are possible. Some of those aspects and embodiments are described below. After reading this specification, skilled artisans will appreciate that those aspects and embodiments are only illustrative and do not limit the scope of the present invention.

In a first aspect, a magnetic resonance imaging system can include a primary magnet, wherein an opening extends through a center of the primary magnet. The magnetic resonance imaging system can also include an analyzing region disposed within the opening, wherein the analyzing region is configured to allow a patient to sit within the magnetic resonance imaging system when the patient is being analyzed.

In one embodiment of the first aspect, the magnetic resonance imaging system is configured, such that it has a single opening for ingress and egress of the patient. In another embodiment, a central axis of the primary magnet lies along a line that is not substantially parallel to a plane corresponding to a floor over which the magnetic resonance imaging system lies. In a particular embodiment, the line intersects the plane at an angle in a range of 30° to 90°. In still another embodiment, a central axis of the primary magnet lies along a line that is substantially perpendicular to a plane corresponding to a floor over which the magnetic resonance imaging system lies.

In a further embodiment of the first aspect, the magnetic resonance imaging system further includes a gradient coil, wherein the primary magnet has a first type of shape, and the gradient coil has a second type of shape different from the first type of shape. In still a further embodiment, the magnetic resonance imaging system further includes a seat, a chair, or a combination thereof, wherein the seat, the chair, or the combination is designed to be partly or completely disposed within the analyzing region during a typical operation of the magnetic resonance imaging system.

In a second aspect, a method of using a magnetic resonance imaging system can include disposing a patient within the magnetic resonance imaging system, wherein the magnetic resonance imaging system includes a primary magnet having an annulus, and an opening disposed within the annulus. The method can also include analyzing the patient using the magnetic resonance imaging system, wherein analyzing is performed when the patient is disposed within the opening and not in a lying position.

In one embodiment of the second aspect, the method further includes positioning the patient on a seat. In another embodiment, disposing the patient includes placing a combination of the patient and a seat into an analyzing region of the magnetic resonance imaging system. In still another embodiment, during analyzing the patient, a head and a leg of the patient extend partly or completely outside of a same side of the opening within the annulus of the primary magnet.

In a further embodiment of the second aspect, during analyzing the patient, a head and a leg of the patient partly or completely extend outside of an analyzing region of the magnetic resonance imaging system. A first line includes a first point corresponding to a center of a pelvic region of the patient and a second point corresponding to a center at a base of a neck of the patient, and a second line includes the first point and a third point corresponding to a center of a knee of the patient. An angle is formed at an intersection of the first line and the second line, wherein the angle is no greater than 120°. In still a further embodiment, the method further includes contacting the patient with an object, wherein contacting is performed when the patient and the object are disposed within the analyzing region and when a primary magnet of the magnetic resonance imaging system is at a magnetic field significantly greater than 0.0 T.

In a third aspect, a magnetic resonance imaging system can include a primary magnet having a first type of shape and a gradient coil having a second type of shape different from the first type of shape.

In one embodiment of the third aspect, the first type of shape includes an annulus, and the second type of shape includes an outer perimeter and is substantially solid at all points within the outer perimeter. In another embodiment, the first type of shape includes a substantially open, substantially cylindrical shape, and the second type of shape includes a substantially flat, substantially circular shape. In a particular embodiment, the first type of shape includes a first opening having a first opening width, and the second type of shape includes a second opening having a second opening width, wherein the first opening width is larger than the second opening width.

In a fourth aspect, a gradient coil for a magnetic resonance imaging system can include a substantially cylindrical portion including an annulus and a first opening within the annulus, and a substantially flat portion adjacent to the substantially cylindrical portion, wherein the substantially flat portion is disposed along a bottom of the first opening.

In one embodiment of the fourth aspect, the substantially cylindrical portion abuts the substantially flat portion. In another embodiment, the first opening of the substantially cylindrical portion has a first opening width, and the substantially flat portion further includes an outer perimeter that has a corresponding outer perimeter width, wherein the corresponding outer perimeter width is no greater than 3 times larger than the first opening width. In still another embodiment, the substantially flat portion further includes a second opening that has a second opening width, wherein the first opening width is larger than the second opening width.

Note that not all of the activities described above in the general description or the examples are required, that a portion of a specific activity may not be required, and that one or more further activities may be performed in addition to those described. Still further, the order in which activities are listed is not necessarily the order in which they are performed.

According to further embodiments, a magnetic resonance imaging system includes a primary magnet, wherein an opening extends through a center of the primary magnet, and an analyzing region disposed within the opening, wherein the analyzing region is configured to allow a patient to sit within the magnetic resonance imaging system when the patient is being analyzed. Additionally, it can be possible, according to a further embodiment, that the magnetic resonance imaging system can be configured, such that it has a single opening for an ingress and an egress of the patient. Additionally or alternatively, a central axis of the primary magnet can lie along a line that is not substantially parallel to a plane corresponding to a floor over which the magnetic resonance imaging system lies. Thereby, as one example, the line can intersect the plane at an angle in a range of 30° to 90°. According to further embodiments, which may be combined with other embodiments described herein, a central axis of the primary magnet can lie along a line that is substantially perpendicular to a plane corresponding to a floor over which the magnetic resonance imaging system lies. According to yet further embodiments, alternatively or additionally, the magnetic resonance imaging system can include a gradient coil, wherein the primary magnet has a first type of shape, and the gradient coil has a second type of shape different from the first type of shape. According to even further embodiments, which can be combined with other embodiments described herein, the magnetic resonance imaging system may further include a seat, a chair, or a combination thereof, wherein the seat, the chair, or the combination is designed to be disposed within the analyzing region during a typical operation of the magnetic resonance imaging system.
According to embodiments described herein, a method of using a magnetic resonance imaging system includes disposing a patient within the magnetic resonance imaging system, wherein the magnetic resonance imaging system comprises a primary magnet having an annulus, and an opening disposed within the annulus, and analyzing the patient using the magnetic resonance imaging system, wherein analyzing is performed when the patient is disposed within the opening and not in a lying position. Thereby, as an example, it is possible to position the patient on a seat. As one typical aspect, disposing the patient can include placing a combination of the patient and a seat into an analyzing region of the magnetic resonance imaging system.
According to another embodiment, during analyzing the patient, a head and a leg of the patient may extend outside of a same side of the opening within the annulus of the primary magnet. Additionally or alternatively, during analyzing the patient, a head and a leg of the patient can extend outside of an analyzing region of the magnetic resonance imaging system, a first line can include a first point corresponding to a center of a pelvic region of the patient and a second point corresponding to a center at a base of a neck of the patient; a second line can include the first point and a third point corresponding to a center of a knee of the patient, and an angle can be formed at an intersection of the first line and the second line, wherein the angle is no greater than 120°. According to yet further embodiments, which can be combined with other embodiments described herein, the method may further include contacting the patient with an object, wherein contacting is performed when the patient and the object are disposed within the analyzing region and when a primary magnet of the magnetic resonance imaging system is at a magnetic field significantly greater than 0.0 T.
According to further embodiments, a magnetic resonance imaging system includes a primary magnet having a first type of shape, and a gradient coil having a second type of shape different from the first type of shape. Typically, the first type of shape can include an annulus, and the second type of shape can include an outer perimeter and is substantially solid at all points within the outer perimeter. Additionally or alternatively, the first type of shape can include a substantially open, substantially cylindrical shape, and the second type of shape can include a substantially flat, substantially circular shape. Further additionally or alternatively, the first type of shape can include a first opening having a first opening width, and the second type of shape can include a second opening having a second opening width, wherein the first opening width is larger than the second opening width.
According to further embodiments, a gradient coil for a magnetic resonance imaging system includes a substantially cylindrical portion including an annulus and a first opening within the annulus, and a substantially flat portion adjacent to the substantially cylindrical portion, wherein the substantially flat portion is disposed along a bottom of the first opening. Thereby, as a typical embodiment, the substantially cylindrical portion can abut the substantially flat portion. According to another embodiment, which can be combined with other embodiments described herein, the first opening of the substantially cylindrical portion can have a first opening width, and the substantially flat portion further can include an outer perimeter that has a corresponding outer perimeter width, wherein the corresponding outer perimeter width is no greater than 3 times larger than the first opening width. As a further example, the substantially flat portion further can include a second opening that has a second opening width, wherein the first opening width is larger than the second opening width.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that a structural substitution, logical substitution, or another change may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. § 1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed subject matter requires more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges includes each and every value within that range.

The above-disclosed subject matter is to be considered illustrative, and not restrictive, and the appended claims are intended to cover any and all such modifications, enhancements, and other embodiments that fall within the scope of the present invention. Thus, to the maximum extent allowed by law, the scope of the present invention is to be determined by the broadest permissible interpretation of the following claims and their equivalents, and shall not be restricted or limited by the foregoing detailed description.

## Claims

1. A magnetic resonance imaging system (100) comprising:
a primary magnet (102; 202), wherein an opening (226) extends through a center of the primary magnet; and
an analyzing region disposed within the opening, wherein the analyzing region is configured to allow a patient to sit within the magnetic resonance imaging system when the patient is being analyzed.

2. The magnetic resonance imaging system of claim 1, wherein the magnetic resonance imaging system is configured, such that it has a single opening for an ingress and an egress of the patient.

3. The magnetic resonance imaging system of any of claims 1 to 2, further comprising a gradient coil (104; 304; 404), wherein:
the primary magnet has a first type of shape; and
the gradient coil has a second type of shape different from the first type of shape.

4. The magnetic resonance imaging system of claim 3, wherein:
the first type of shape includes an annulus (224); and
the second type of shape includes an outer perimeter and is substantially solid at all points within the outer perimeter.

5. The magnetic resonance imaging system of any of claims 3 to 4, wherein:
the first type of shape includes a substantially open, substantially cylindrical shape; and
the second type of shape includes a substantially flat, substantially circular shape.

6. The magnetic resonance imaging system of any of claims 3 to 5, wherein:
the first type of shape includes a first opening (226) having a first opening width; and
the second type of shape includes a second opening (326; 426) having a second opening width, wherein the first opening width is larger than the second opening width.

7. A method of using a magnetic resonance imaging system (100), the method comprising:
disposing a patient within the magnetic resonance imaging system, wherein the magnetic resonance imaging system comprises a primary magnet (102; 202) having an annulus, and an opening (226) disposed within the annulus; and
analyzing the patient using the magnetic resonance imaging system, wherein analyzing is performed when the patient is disposed within the opening and not in a lying position.

8. The method of claim 7, wherein disposing the patient comprises placing a combination of a patient and a seat (149) into an analyzing region of the magnetic resonance imaging system.

9. The method of any of claims 7 to 8, wherein, during analyzing the patient, a head and a leg of the patient extend outside of a same side of the opening within the annulus of the primary magnet.

10. The method of any of claims 7 to 9, further comprising contacting the patient with an object, wherein contacting is performed when the patient and the object are disposed within the analyzing region and when a primary magnet of the magnetic resonance imaging system is at a magnetic field significantly greater than 0.0 T.
